# EUROPÄISCHE PATENTSCHRIFT

(11) **EP 0 519 903 B1**
(45) Veröffentlichungstag und Bekanntmachung des Hinweises auf die Patenterteilung: **25.09.2002**
(21) Anmeldenummer: 92890147.9
(22) Anmeldetag: 16.06.1992
(51) Int. Cl.: A61K 38/46, A61K 38/54

(54) **Arzneimittel enthaltend aktiviertes Protein C**
Drug containing activated protein C
Médicament contenant de la protéine C activée

(30) Priorität: 20.06.1991 AT 123991
(43) Veröffentlichungstag der Anmeldung: 23.12.1992
(73) Patentinhaber: Baxter Aktiengesellschaft, 1221 Wien (AT)
(72) Erfinder: Eibl, Johann, Dr., A-1180 Wien (AT); Linnau, Yendra, Dr., A-1224 Wien (AT); Philapitsch, Anton, A-2490 Ebenfurt (AT); Schwarz, Hans Peter, Doz. Dr., A-1180 Wien (AT)
(74) Vertreter: Alge, Daniel, Mag. Dr. rer.nat.

(56) Entgegenhaltungen:
- EP-A- 0 287 028
- EP-A- 0 318 201
- WO-A-90/07524
- ADVANCES IN APPLIED BIOTECHNOLOGY SERIES Bd. 11, 1990, Seiten 83 - 89; H. SCHWARZ ET AL: 'Monoclonal antibody purified protein C concentrate: Initial clinical experience'

## Beschreibung

Die Erfindung betrifft ein Verfahren zur Herstellung ein parenteral verabreichbaren Arzneimittels mit thrombolytischer Wirkung enthaltend aktiviertes Protein C, welches frei ist von Thrombinaktivität und keine Antikörper gegen Protein C oder aktiviertes Protein C enthält.

Diese Arzneimittel können als Thrombolytikum, Fibrinolytikum und als Antikoagulans sowie zur Behandlung von Protein C-Mangelzuständen angewendet werden.

Protein C ist ein Vitamin-K-abhängiges Glykoprotein, das in der Leber synthetisiert wird und im Plasma in einer Konzentration von 4 µg/ml als inaktives Zymogen zirkuliert. Es wird an der Gefäßwandoberfläche (Endothel) durch den Thrombin-Thrombomodulinkomplex in die aktive Serinprotease (aktiviertes Protein C) übergeführt. Es ist bekannt, daß aktiviertes Protein C profibrinolytische Eigenschaften hat. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Die Aktivierung von Protein C in vivo stellt eine negative Feedback-Reaktion der Thrombingenerierung dar. Um optimale biologische Aktivität zu entfalten, ist ein Kofaktor (Protein S) notwendig.

In der PCT-Veröffentlichung WO 90/12028 sowie in der EP-A - 0 287 028 werden Verfahren zur Aktivierung von Protein C und die affinitätschromatographische Reinigung beschrieben. Die Reaktion des Protein C mit Thrombin/Thrombomodulin wird gemäß EP-A - 0 287 028 durch Zugabe von Antithrombin III gestoppt.

Bei der Verabreichung von affinitätschromatographisch an nicht sterilen monoklonalen Antikörpern gereinigten Protein C-Präparaten besteht die Gefahr der Übertragung von Krankheitserregern. Weiters können sich Verunreinigungen, wie Thrombinspuren, Spuren von murinen Proteinen oder Serumamyloid P bei Verabreichung von Präparaten enthaltend aktiviertes Protein C nachteilig auswirken.

Zur Erhöhung der Virussicherheit von Protein C-Präparaten wird in "Advances in Applied Biotechnology Series" Vol. 11, "Protein C and Related Anticoagulants" (Bruley D.F. and Drohan W.N. eds), Gulf Publishing Company, Houston, London, Paris, Zürich, Tokyo, pp 83-89 (1990) die Isolierung des Protein C aus einem bereits virusinaktivierten Ausgangsmaterial durch affinitätschromatographische Reinigung an virusinaktivierten monoklonalen Antikörpern vorgeschlagen. Das Präparat kann anschließend erneut einer Behandlung zur Virusinaktivierung unterworfen werden.

Aus der PCT-Veröffentlichung WO 90/07524 ist bekannt, daß Serumamyloid P durch Adsorption des Protein C an einem Ionenaustauscher (Sephadex Q50) entfernt werden kann.

Aktiviertes Protein C kann weiters durch selektive Adsorption des Thrombins an Heparin-Sepharose von Thrombinspuren befreit werden (Thromb. Res. 59, 27-35 (1990)). Dieses Verfahren umfaßt jedoch keinen Schritt zur Virusinaktivierung und zur Entfernung von Serumamyloid P.

In der EP-A - 0 318 201 wird beschrieben, daß aktiviertes Protein C alleine oder in Kombination mit einer thrombolytisch wirksamen Substanz zur Verhinderung arterieller Thrombosen oder Thromboembolie eingesetzt werden kann. Beispiele zeigen die antithrombotische Wirkung von aktiviertem Protein C.

Die Erfindung stellt sich die Aufgabe, ein Arzneimittel der eingangs beschriebenen Art zur Verfügung zu stellen, das ein besonders reines aktiviertes Protein C enthält.

Das erfindungsgemäße Arzneimittel ist dadurch gekennzeichnet, daß es durch Aktivierung von Protein C erhalten wird und frei ist von Serumamyloid P und von infektiösen Agentien.

Das erfindungsgemäße Arzneimittel besitzt keine Nebenwirkungen: die Thrombolysetherapie ruft keinen Fibrinogenabfall oder Blutungen hervor.

Es hat sich gezeigt, daß das erfindungsgemäße Arzneimittel insbesondere in Kombination mit Lys-Plasminogen oder Urokinase unerwartet gut thrombolytisch wirkt und in größerem Ausmaß Thrombosen verhindern kann.

Die Erfindung beruht auf der Erkenntnis, daß die Verabreichung von aktiviertem Protein C im Organismus zu einer Freisetzung des thrombolytisch wirksamen Enzyms Plasmin führt und somit zu einer gesteigerten thrombolytischen Aktivität in vivo. Das erfindungsgemäße Arzneimittel eignet sich daher als Thrombolytikum. Es hat sich weiters gezeigt, daß die thrombolytische Wirkung dosisabhängig ist und daß sie sich nur dann optimal entfaltet, wenn das verwendete aktivierte Protein C von Thrombinaktivität und von Serumamyloid P befreit ist.

Die Erfindung betrifft demgemäß ein Verfahren zur Herstellung des oben beschriebenen Arzneimittels, welches Verfahren gekennzeichnet ist durch die Kombination der Maßnahmen, daß
- Protein C affinitätschromatographisch mit Anti-Protein C-Antikörpern, welche gegebenenfalls gegen infektiöse Agentien inaktiviert sind, gereinigt wird,
- das gereinigte Protein C mit Thrombin aktiviert und das Thrombin anschließend inhibiert wird,
- das aktivierte Protein C chromatographisch mit Ionenaustauschern von Serumamyloid P und von Antikörpern befreit,
und zu einem parenteral verabreichbaren Arzneimittel aufgearbeitet wird.

Eine bevorzugte Ausführungsform des erfindungsgemäßen Verfahrens ist dadurch gekennzeichnet, daß mindestens zwei Maßnahmen zur Inaktivierung infektiöser Agentien vorgesehen werden.

Das erfindungsgemäße Arzneimittel enthält aktiviertes Protein C in besonders reiner Form. Thrombin wird aus dem Reaktionsgemisch am besten mit 0,01 bis 0,1 IE Antithrombin III in Form eines Antithrombin III-Heparin-Komplexes pro Einheit Thrombin inhibiert und anschließend entfernt. Der Antithrombin III-Heparin-Komplex weist eine erheblich höhere Affinität zum Thrombin auf als Antithrombin III alleine, weshalb die Abtrennung des Thrombins von aktiviertem Protein C besser möglich ist.

Abgesehen von der Freiheit von Thrombinspuren ist das erfindungsgemäße Arzneimittel durch eine chromatographische Reinigung an einem Ionenaustauscher auch von murinen Proteinen (Antikörpern) und Serumamyloid P befreit und ist überdies durch die Kombination mehrerer Behandlungen zur Virusinaktivierung als virussicher anzusehen. Zur Gewinnung des Zymogens kann bereits virusinaktiviertes Ausgangsmaterial (Prothrombinkomplex) affinitätschromatographisch an virusinaktivierten monoklonalen Antikörpern gereinigt werden. Nach Aktivierung mit Thrombin kann das aktivierte Protein C erneut einer Behandlung zur Virusinaktivierung unterzogen werden. Zur Inaktivierung humanpathogener Keime können bekannte Inaktivierungsverfahren herangezogen werden (z.B. EP-A-0 159 311, EP-A-0 050 061, EP-A-0 131 740).

Vorteilhaft wird die Inhibierung von Thrombin durch Zugabe von Antithrombin III-Heparin-Komplex vorgenommen.

Im folgenden wird die Herstellung einer aktivierten Protein C-hältigen pharmazeutischen Präparation und ihre im Tiermodell zu beobachtende thrombolytische Wirkung näher beschrieben.

### Gewinnung von Protein C

Reines Protein C wurde aus einer rohen Protein C-Fraktion gewonnen, die aus kommerziell erhältlichem Prothrombinkomplex-Konzentrat hergestellt wurde. Die Reinigung erfolgte affinitätschromatographisch mittels monoklonaler Antikörper. Monoklonale Anti-Protein C-Antikörper wurden wie folgt hergestellt:

BALB/C-Mäuse wurden mit 100 µg menschlichem Protein C in zweiwöchigen Abständen durch intraperitoneale Injektion immunisiert. Nach sechs Wochen wurden noch einmal 50 µg des menschlichen Protein C injiziert und drei Tage später die Fusion vorgenommen. Die Myelomzellinie (P3-X-63-AG8-653, 1,5 x 10⁷ Zellen) wurde vermischt mit 1,7 x 10⁸ Milzzellen von einer Maus, und die Fusion nach modifizierter Köhler & Milstein-Methode unter Verwendung von PEG 1500 durchgeführt (Köhler G., Milstein C., Nature 256(1975)495-497).

Positive Klone, getestet mittels eines ELISA, wurden zweimal subgeklont. Aszitesproduktion erfolgte durch Injektion von 5 x 10⁶ Hybridomzellen je BALB/C-Maus zwei Wochen nach Pristan-Behandlung.

Das Immunglobulin wurde aus Aszites durch Ammoniumsulfatpräzipitation und anschließende Chromatographie mittels QAE-Sephadex und darauf folgender Chromatographie an Sephadex G200 gereinigt. Um das Risiko der Übertragung muriner Viren zu reduzieren, wurde der Antikörper vor der Immobilisierung noch einem Virusinaktivierungsschritt unterworfen. Die so erhaltenen monoklonalen Antikörper gegen Protein C wurden an CNBR-Sepharose 4B (Pharmacia) gekoppelt. Für die Reinigung des Protein C mittels Affinitätschromatographie wurden folgende Puffer verwendet:

Als Adsorptionspuffer: 20 mM Tris, 2 mM EDTA, 0,25 M NaCl und 5 mM Benzamidin;
als Waschpuffer wurde verwendet: 20 mM Tris, 1 M NaCl, 2 mM Benzamidin, 2 mM EDTA; der pH-Wert betrug 7,4; als Elutionspuffer wurde verwendet: 3 M NaSCN, 20 mM Tris, 1 M NaCl, 0,5 mM Benzamidin, 2 mM EDTA.

Im einzelnen: Das Prothrombinkomplex-Konzentrat wurde im Adsorptionspuffer aufgelöst, wobei etwa 10 g des Prothrombinkomplex-Konzentrates für eine 20 ml monoklonale Antikörpersäule zur Verwendung kamen. Anschließend wurde das aufgelöste Prothrombinkomplex-Konzentrat filtriert, bei 20.000 rpm 15 min lang zentrifugiert und durch ein 0,8 µm Filter sterilfiltriert. Das sterilfiltrierte und gelöste Prothrombinkomplex-Konzentrat wurde auf die Säule aufgetragen mit einer Flußrate von 10 ml/h. Anschließend wurde die Säule mit dem Waschpuffer proteinfrei gewaschen und schließlich das gebundene Protein C mit dem Elutionspuffer bei einer Flußrate von 5 ml/h eluiert und die Fraktionen gesammelt. Das eluierte Protein C wurde gegen einen Puffer (0,2 M Tris, 0,15 M Glycin und 1 mM EDTA, pH 8,3) dialysiert. Der Protein C-Gehalt wurde antigenmäßig mittels der Methode nach Laurell und aktivitätsmäßig nach Protac-Aktivierung bestimmt.

Die erhaltene, Protein C-hältige Lösung wurde sterilfiltriert, lyophilisiert und durch einstündige Dampfbehandlung bei 80° C ± 5° C und 1375 ± 35 mbar virusinaktiviert.

### Gewinnung von aktiviertem Protein C

Die Aktivierung von gereinigtem Protein C erfolgte dadurch, daß 10 g Protein C in einem Puffer (150 mM NaCl, 15 mM Natrium-citrat, pH = 7,5) in einer Konzentration von 6 mg/ml gelöst wurden und mit 14,4 mg humanem, virusinaktiviertem Thrombin, welches in einem 100 mM NaClund 10 mM Natriumcitrat-Puffer gelöst war, drei Stunden bei 37°C inkubiert wurden. Die Reaktion wurde mit 1.200 E Antithrombin III-Heparin-Komplex gestoppt.

Anschließend erfolgte die Weiterreinigung des aktivierten Protein C an Q-Sepharose. Dazu wurde die Adsorption in 150 mM NaCl und 15 mM Natriumcitrat durchgeführt; eluiert wurde mit 1 M NaCl-Lösung.

Das Eluat wurde ultrafiltriert, sterilfiltriert, in Einheiten zu je 5 ml steril abgefüllt und lyophilisiert.

Nach Auflösung zu einem Volumen von 5 ml hatte das Präparat folgende Zusammensetzung:

| | |
|---|---|
| Protein | 1,5 mg/ml |
| aPC | 204 E/ml (amidolytisch mit dem chromogenen Substrat S2366 von Fa. Kabi bestimmt) |
| NaCl | 150 mM |
| Natriumcitrat | 15 mM |
| pH | 7,5 |

Das Präparat war frei von Antikörpern gegen Protein C und Thrombin (immunologisch bestimmt) und weiters frei von Serumamyloid P (elektrophoretisch bestimmt).

### Tiermodell

Die thrombolytische Eigenschaft von menschlichem aktiviertem Protein C wurde in einem in der Literatur bekannten und zur Testung thrombolytischer Substanzen als Standard angesehenen Modell evaluiert. Das von D. Collen 1983 beschriebene Modell wurde modifiziert, indem statt ¹²⁵I Fibrinogen ¹²³Human Fibrinogen verwendet wurde und eine Gammakamera zum Einsatz kam (Fa. Elscint APEX 409AG, Single Pinhole Collimator).

Die linke Jugularvene von mit Hypnorm (Janssen) anästhesierten Kaninchen wurde freipräpariert und ein Katheter in den Ramus facialis eingebunden. Nach Abklemmen der Jugularvene auf eine Distanz von etwa 1,5 cm wurde durch den Katheter 100 µl eines ¹²³I Fibrinogen-Kaninchenblutgemisches appliziert. Nach 30 Minuten wurden die Klammern entfernt. aPC wurde über die Femoralvene über 1 Stunde infundiert. Die Gammakamera wurde über den ¹²³ Fibrinogenthrombus in einer Distanz von 2 cm plaziert. Die Radioaktivität wurde 2 Stunden lang gemessen und kontinuierlich aufgezeichnet. Die Werte wurden um den Zerfall des ¹²³I Fibrinogens korrigiert. Die Thrombolyserate wurde als Abnahme der Radioaktivität im Thrombus ausgedrückt.

In jeder Dosierungsgruppe wurden 4 Tiere verwendet. Eine Gruppe wurde mit Coumarin (4-Hydroxycoumarin, Sigma, 5 mg/kg i.v. über 9 Tage) vorbehandelt, um die Synthese Vitamin-K-abhängiger Gerinnungsfaktoren auszuschalten. Der Thrombus bei den antikoagulierten Tieren wurde mit einem normalen, d.h. nicht antikoagulierten, Kaninchenblut-Fibrinogengemisch hergestellt.

Die Infusion von aPC führte zu einer dosisabhängigen Thrombolyse: 80, 150, 200 und 500 E/kg führten zu 21, 24, 40 und 58 % Thrombolyse. Mit Puffer behandelte Tiere zeigten im gleichen Meßzeitraum von 2 Stunden lediglich 12 % Lyse. Die antikoagulierten Tiere waren Lyse-resistent und zeigten mit 400 E/kg nur 18 % Lyse. Dies weist darauf hin, daß Vitamin K-abhängige Proteine (z.B. Protein S, Protein Z, Prothrombin) für den thrombolytischen Effekt von aPC in vivo notwendig sind.

Diese Ergebnisse sind im Diagramm graphisch dargestellt, wobei als Abszisse die Dosis (Einheiten/kg) und als Ordinate der thrombolytische Effekt (% Lyse) gewählt wurden.

Es hat sich gezeigt, daß aPC besser zur Auflösung venöser Thromben geeignet ist als eine Kombination von aPC mit t-PA. Bei Verabreichung von 500 Einheiten aPC/kg und 0,4 mg t-PA/kg konnte lediglich eine Lyse von etwa 33 % beobachtet werden (verglichen mit 58 % bei alleiniger Anwendung von aPC).

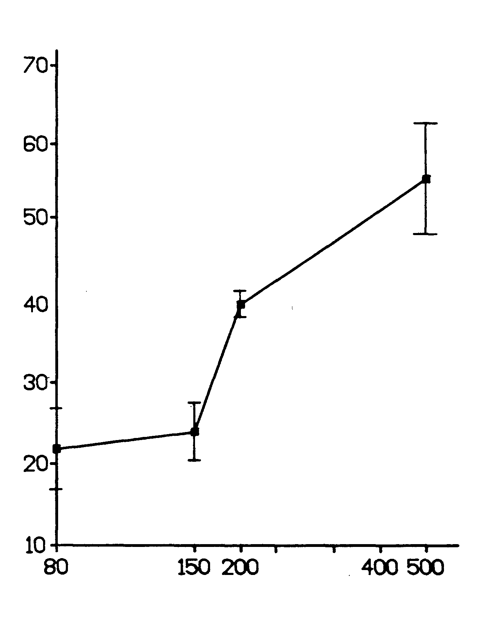

## Patentansprüche

1. Verfahren zur Herstellung eines parenteral verabreichbaren Arzneimittels mit thrombolytischer Wirkung enthaltend aktiviertes Protein C, welches frei ist von Thrombinaktivität und keine Antikörper gegen Protein C oder aktiviertes Protein C enthält und welches frei ist von Serumamyloid P und von infektiösen Agentien, **gekennzeichnet durch** die Kombination der Maßnahmen, daß
- Protein C affinitätschromatographisch mit Anti-Protein C-Antikörpern, welche gegebenenfalls gegen infektiöse Agentien inaktiviert sind, gereinigt wird,
- das gereinigte Protein C mit Thrombin aktiviert und das Thrombin anschließend inhibiert wird,
- das aktivierte Protein C chromatographisch mit Ionen austauschern von Serumamyloid P und von Antikörpern befreit, und zu einem parenteral verabreichbaren Arzneimittel aufgearbeitet wird.

2. Verfahren nach Anspruch 1, **dadurch gekennzeichnet, daß** mindestens zwei Maßnahmen zur Inaktivierung infektiöser Agentien vorgesehen werden.

3. , Verfahren nach einem der Ansprüche 1 oder 2, **dadurch gekennzeichnet, daß** die Inhibierung von Thrombin durch Zugabe von Antithrombin III-Heparin-Komplex vorgenommen wird.

4. Verwendung von aktiviertem Protein C, gegebenenfalls in Kombination mit Lys-Plasminogen oder Urokinase, zur Herstellung eines Arzneimittels zur Thrombolyse.

5. Verwendung von aktiviertem Protein C, gegebenenfalls in Kombination mit Lys-Plasminogen oder Urokinase, zur Herstellung eines Arzneimittels zum Auflösen venöser Thromben.

## Claims

1. A method of producing a parenterally administrable drug having thrombolytic activity and containing activated protein C, which is free of thrombin activity and does not comprise antibodies to protein C or activated protein C and which is free of serum amyloid P and of infectious agents, **characterised by** the combination of the measures that
• protein C is purified by way of affinity chromatography with anti-protein C antibodies optionally inactivated against infectious agents,
• the purified protein C is activated with thrombin and the thrombin subsequently is inhibited,
• the activated protein C is chromatographically freed from serum amyloid P and from antibodies by aid of ion exchangers and is processed to a parenterally administrable drug.

2. A method according to claim 1, **characterised in that** at least two measures for inactivating infectious agents are provided.

3. A method according to any one of claims 1 or 2, **characterised in that** the inhibition of thrombin is effected by admixing anti-thrombin III-heparin complex.

4. The use of activated protein C, optionally in combination with Lys plasminogen or urokinase, for preparing a drug for thrombolysis.

5. The use of activated protein C, optionally in combination with Lys plasminogen or urokinase, for preparing a drug for dissolving venous thrombi.

## Revendications

1. Procédé de préparation d'un médicament ayant un effet thrombolytique et contenant de la protéine C activée, pouvant être administré par voie parentérale, qui est dépourvu d'activité de la thrombine et ne contient pas d'anticorps dirigés contre la protéine C ou la protéine C activée et qui est dépourvu d'amyloïde P sérique et d'agents infectieux, **caractérisé par** la combinaison d'opérations suivantes :
- la protéine C est purifiée par chromatographie d'affinité avec des anticorps anti-protéine C qui sont le cas échéant inactivés contre des agents infectieux,
- la protéine C purifiée est activée avec de la thrombine et ensuite la thrombine est inhibée,
- la protéine C activée est débarrassée de l'amyloïde P sérique et des anticorps par chromatographie sur résines échangeuses d'ions et conditionnée sous la forme d'un médicament pouvant être administré par voie parentérale.

2. Procédé selon la revendication 1, **caractérisé en ce qu'**il est prévu au moins deux opérations pour inactiver les agents infectieux.

3. Procédé selon l'une des revendications 1 ou 2, **caractérisé en ce que** l'inhibition de la thrombine se fait par ajout de complexe antithrombine III-héparine.

4. Utilisation de protéine C activée, le cas échéant en combinaison avec du plasminogène Lys ou de l'urokinase, pour la préparation d'un médicament destiné à la thrombolyse.

5. Utilisation de protéine C activée, le cas échéant en combinaison avec du plasminogène Lys ou de l'urokinase, pour la préparation d'un médicament destiné à la dissolution de thrombus veineux.
